# EUROPEAN PATENT APPLICATION

(11) **EP 1 132 484 A2**
(43) Date of publication of application: **12.09.2001**
(21) Application number: 01105812.0
(22) Date of filing: 08.03.2001
(51) Int. Cl.: C12Q 1/68

(54) **Method for testing complementation of nucleic acid**

(30) Priority: 08.03.2000 JP 2000063129
(71) Applicant: FUJI PHOTO FILM CO., LTD., Kanagawa-ken, 250-0123 (JP)
(72) Inventor: Makino, Yoshihiko, Asaka-shi, Saitama, 351-8585 (JP); Abe, Yoshihiko, Asaka-shi, Saitama, 351-8585 (JP); Ogawa, Masashi, c/o Fuji Photo Film Co., Ltd., Tokyo, 106-8620 (JP); Takagi, Makoto, Fukuoka-shi, Fukuoka, 816-0076 (JP); Takenaka, Shigeori, Koga-shi, Fukuoka, 811-3114 (JP); Yamashita, Kenichi, Fukuoka-shi, Fukuoka, 814-0152 (JP)
(74) Representative: Albrecht, Thomas, Dr.

(57) **Abstract**

A method of for testing complementation of nucleic acid fragment is performed by the steps of:
bringing a sample nucleic acid complex composed of a double-stranded nucleic acid structure and a labeled intercalator, in which the double-stranded nucleic acid structure has been produced by contact of a sample nucleic acid fragment with a probe molecule fixed to a solid carrier, into contact with an aqueous medium;
applying variation of surrounding conditions to the aqueous medium, to cause disengagement of the sample fragment and intercalator from the complex and simultaneously measuring decrease of quantity of the label on the carrier, so that stability of the sample fragment in the complex is determined; and
comparing the stability determined above with reference stability data.

## Description

### FIELD OF THE INVENTION

This invention relates to a method for testing complementation of a nucleic acid fragment. In particular, the invention relates to detection of a partly complementary nucleic acid fragment. The detection of a partly complementary nucleic acid fragment is of great value in the gene analyses such as analysis of gene polymorphism and analysis of variation of abnormal gene.

### BACKGROUND OF THE INVENTION

The gene analysis is recently paid an attention in developing gene technology.

As a representative gene analysis method for detecting variation of gene fragments, SSCP (i.e., single-stranded conformation polymorphism) is known. In the SSCP procedure, each of double stranded normal gene fragments and double stranded abnormal gene fragments (which is different from the normal gene fragment in the base unit positioned in the crossed place) is treated to give a set of two single stranded gene fragments. Both of the single stranded gene fragments are subjected to electrophoresis on a polyacrylamide gel. Each gene fragment has a specific high-order structure differing from each other. Accordingly, each of the single stranded gene fragments moves differently from each other in the electrophoresis. The detection of the movement of the gene fragment gives an information of variation of gene fragments.

The SSCP method, however, sometimes fails to differentiate an abnormal gene fragment from a normal gene fragment, because a certain abnormal gene fragment moves similarly to the corresponding normal gene fragment. Particularly, if the variation of base takes place in the vicinity of a gene fragment, it is likely that the abnormal gene fragment takes almost the same high-order structure as that of the corresponding normal gene fragment, and hence that there is caused almost no difference of movement in the electrophoresis.

The variation of gene fragment from a single stranded normal gene fragment to a single stranded abnormal gene fragment can be also detected by way of hybridization. In the hybridization, a hybrid structure is formed differently between the normal gene fragment and the abnormal gene fragment.

Japanese Patent PCT Publication H9-501561 describes detection of a double-stranded nucleic acid hybrid having a specific structure by means of a fluorescent microscope.

It is also known that different double-stranded nucleic acid hybrids can be detected by temperature graduation gel electrophoresis.

It is known that there are three types of hybrid structures, namely,
a full-match structure in which two single stranded DNA fragments are hybridized to form a complete double-stranded DNA fragment;
a mismatch structure in which two single stranded DNA fragments are hybridized to form a double-stranded DNA fragment having one uncoupled base unit, which is generally observed in the case that a normal DNA fragment is hybridized with an abnormal DNA fragment which differs from the normal DNA fragment in one base unit; and
a bulge structure in which two single stranded DNA fragments are hybridized to form a double-stranded DNA fragment having two or more uncoupled base units.

Japanese Patent Provisional Publication H9-288080 describes a method for detecting a double stranded DNA fragment of a full-match structure by way of an electrochemical detection method. In this method, a sample DNA fragment is electrochemically detected using a DNA probe which is complementary to the sample DNA fragment in the presence of an electrochemically active thread intercalator.

P.E. Nielsen et al., Science, 254, 1497-1500(1991) and P.E. Nielsen et al., Biochemistry, 36, pp.5072-5077 (1997) describe PNA (Peptide Nucleic Acid or Polyamide Nucleic Acid) which has no negative charge and functions in the same manner as DNA does. PNA has a polyamide skeleton of N- (2-aminoethyl)glycine units and has neither glucose units nor phosphate groups.

Since PNA is electrically neutral and is not charged in the absence of an electrolytic salt, PNA is able to hybridize with a complementary nucleic acid to form a hybrid which is more stable than hybrid given by a DNA prove and its complementary nucleic acid (Preprint of the 74th Spring Conference of Japan Chemical Society, pp. 1287, reported by Naomi Sugimoto).

Japanese Patent Provisional Publication H11-332595 describes a PNA probe fixed on a electroconductive substrate at its one end and a detection method utilizing the PNA probe. The PNA probe is fixed onto the electroconductive substrate by the avidin-biotin method.

The aforementioned P.E. Nielsen et al., Science, 254, 1497-1500(1991) also describes a PNA probe labelled with isotope element and a detection method of a complementary nucleic acid.

### SUMMARY OF THE INVENTION

It is an object of the present invention to provide a method of analyzing a nucleic acid sample to judge whether the nucleic acid sample has a base sequence which is partly complementary or fully complementary to a DNA fragment or PNA fragment in its specific base sequence. This analyzing method is of great value in the gene analysis such as analysis of gene polymorphism or analysis of variation of abnormal gene.

The present invention resides in a method for testing complementation of nucleic acid fragment which comprises the steps of:
bringing a sample nucleic acid complex which comprises a double-stranded nucleic acid structure and a labeled intercalator intercalated therein, in which the double-stranded nucleic acid structure has been produced by contact of a sample nucleic acid fragment with a probe molecule fixed to a solid carrier in the presence of an aqueous medium, the probe molecule being selected from the group consisting of a nucleic acid or a nucleic acid derivative, into contact with an aqueous medium;
applying variation of physical or chemical surrounding conditions to the latter aqueous medium, to cause disengagement of the sample nucleic acid fragment and the intercalator from the nucleic acid complex and simultaneously measuring decrease of quantity of the label on the solid carrier, so that stability of the sample nucleic acid fragment of the complex is determined; and
comparing the stability determined above with reference stability data which are separately obtained by determination of stability of a reference nucleic acid fragment in a reference nucleic acid complex comprising a reference double-stranded nucleic acid structure and the labeled intercalator intercalated therein in which the reference double-stranded nucleic acid structure is produced by contact of the reference nucleic acid fragment with the probe molecule, the reference nucleic acid fragment being determined in complementation thereof with the probe molecule.

The invention also resides in a method for testing complementation of nucleic acid fragment which comprises the steps of:
bringing a sample nucleic acid fragment into contact with a probe molecule fixed to a solid carrier in the presence of an aqueous medium and a labeled intercalator to produce on the solid carrier a sample nucleic acid complex comprising a double-stranded nucleic acid structure and the labeled intercalator intercalated therein, the probe molecule being selected from the group consisting of a nucleic acid or a nucleic acid derivative, while applying variation of physical or chemical surrounding conditions to the aqueous medium, so that stability of the sample nucleic acid fragment in the complex is determined; and
comparing the stability determined above with reference stability data which are separately obtained by determination of stability of a reference nucleic acid fragment in a reference nucleic acid complex comprising a reference double-stranded nucleic acid structure and the labeled intercalator intercalated therein in which the reference double-stranded nucleic acid structure is produced by contact of the reference nucleic acid fragment with the probe molecule, the reference nucleic acid fragment being determined in complementation thereof with the probe molecule.

### BRIEF DESCRIPTION OF DRAWINGS

Fig. 1 schematically shows formation of a double-stranded nucleic acid structure or formation of a single-stranded nucleic acid which depends on hybridization conditions.

Fig. 2 schematically shows procedures of a typical test method of the invention.

Fig. 3 graphically shows results of differential pulse voltamography data given in the detection of stability of a partly complementary sample nucleic acid in a sample nucleic acid complex, in comparison with stability of a fully complementary reference nucleic acid in a reference nucleic acid complex.

### DETAILED DESCRIPTION OF THE INVENTION

The present invention is based on the finding made by the present inventors in that stability of a hybridized (double-stranded) nucleic acid structure composed of a free nucleic acid fragment and a probe molecule fixed to a solid carrier in an aqueous medium is apparently influenced by physical or chemical surrounding conditions in the medium and in that formation of the hybridized nucleic acid structure and disengagement of the nucleic acid fragment from the hybridized structure can be easily detected by the use of a labeled intercalator which can be intercalated into the hybridized nucleic acid structure. In the test procedure, variation of the label signal of the labeled intercalator on the solid carrier is detected, while the aqueous medium is placed under variation of the physical or chemical surrounding conditions. The signals detected in the hybridized structure containing a sample nucleic acid fragment are compared with the signals detected in the hybridized structure containing a reference nucleic acid fragment whose complementation is already is confirmed.

In the complementation test method of the invention, preferred are set forth below.
(1) The method wherein the variation of physical or chemical surrounding conditions is variation of a temperature of the aqueous medium.
(2) The method wherein the variation of physical or chemical surrounding conditions is variation of electrophoretic potential.
(3) The method wherein the variation of physical or chemical surrounding conditions is variation of ionic strength in the aqueous medium.
(4) The method wherein the labeled intercalator is an intercalator having an electroconductive label or an intercalator having a fluorescent label.
(5) The method wherein the probe molecule contains a chain of a base sequence comprising at least three predetermined base units in series. The reference nucleic acid fragment preferably contains a chain of a base sequence comprising at least three base units in series which are fully complementary to the chain of the probe molecule.
(6) The method wherein the reference stability data are obtained by a step which is identical to the first step of claim 1, except for replacing the sample nucleic acid complex with the reference nucleic acid complex.
(7) The method wherein the probe molecule is selected from the group consisting of oligonucleotide, polynucleotide, and peptide nucleic acid.

### [Detection tool - DNA chip]

The solid carrier having thereon a probe molecule is a known tool for fixing a nucleic acid fragment to the probe molecule fixed by hybridization. This tool is generally called "DNA chip".

Typically, the solid carrier is an electroconductive substrate which is generally employed in the known electrochemical detection method or a non-electroconductive substrate which is generally employed in the known fluorescence detection method.

### [Solid carrier]

The electroconductive substrate (e.g., electrode) may be provided on an electro-insulative support material. The electroconductive substrate and the support material preferably have a less hydrophilic surface or a hydrophobic surface. The electroconductive substrate may have a plain surface or a surface having many fine concaves and convexes.

The electro-insulative support material can be prepared from glass, ceramics, polymer materials (e.g., polyethylene terephthalate, cellulose acetate, polycarbonate of Bisphenol A, polystyrene, poly(methyl methacrylate), silicon, active carbon, and porous materials (e.g., porous glass, porous ceramics, porous silicon, porous active carbon, cloth, knitted cloth, non-woven cloth, filter paper, and membrane filter). Polymer materials, glass, and silicon are preferably employed.

Generally, the electro-insulative support material is employed in the form of a sheet (or a film). The sheet of the support material preferably has a thickness in the range of 100 to 1,000 µm.

The electroconductive substrate can be made of electrode material, optical fiber, photodiode, thermistor, piezo electrical element, or surface elasticity element. The electrode material is generally employed. The electrode can be carbon electrode of graphite or glassy carbon, noble metal electrode of platinum, gold, palladium, or rhodium, metal oxide electrode of titanium dioxide, tin oxide, manganese oxide, or lead oxide, semiconductor electrode of Si, Ge, ZnO, or Cds, or electron conductor of titanium. Preferred are glassy carbon electrode and gold electrode. The electrode may be covered with electroconductive polymer film or monomolecular film.

The DNA chip of the invention is preferably composed of a hydrophobic, electro-insulative support material, a plurality of hydrophobic electroconductive substrates placed on the support material, a plurality of DNA fragments fixed on each of the electroconductive substrates. Each of the electroconductive substrates is preferably arranged apart from the adjoining electroconductive substrates so that each substrate is insulated from the adjoining substrates. The electroconductive substrate may be placed on the support material via an intermediate layer such as a hydrophilic intermediate layer which may have electron charges.

An example of the structure composed of an electro-insulative support material and a plurality of electrodes arranged on the support material is a silicon chip described in Sosnowski, R.G., et al., Proc. Natl. Acad. USA, 94, 1119-1123(1997). The electrode may be produced on a polymer film using a composite sheet of a polymer film and a metal film.

If the fluorescence detection method is employed, the solid carrier can comprise an electro-insulative support material described above.

### [Probe molecule]

The probe molecule to be fixed onto the solid carrier is a nucleic acid (such as DNA fragment, synthesized oligonucleotide, peptide nucleic acid).

As is described before, the test method of the invention is preferably employed for detecting an abnormal gene fragment having one or more different bases from its corresponding normal gene fragment. Accordingly, the normal gene fragment or the abnormal gene fragment is preferably employed as the probe molecule to be fixed on the substrate. It is preferred that the normal gene fragment is used as the DNA probe.

Otherwise, DNA fragment to be fixed onto the substrate may be oligonucleotide, which is favorably employed for studying variations and polymorphism of gene. The DNA fragment to be fixed onto the substrate preferably is one of 3 to 50 mers, more preferably 10 to 25 mers.

If the DNA chip comprises plural DNA chip units each of which has a substrate (e.g., electrode) and probe molecules fixed onto the substrate, the plural DNA chip units can have the same probe molecules or different probe molecules.

Fixation of the probe molecules onto the substrate can be done by any of known methods. For instance, DNA fragments having a reactive group on one end can be fixed onto the substrate through covalent bond by the reaction of the reactive group and the functional group of the surface of the substrate. For instance, a mercapto group is attached to DNA fragment at its 5'- or 3'-terminal, and the mercapto group is then caused to react with a gold electrode, so that a substrate having DNA fragments fixed thereon is produced. The procedure for attaching a mercapto group to DNA fragments is described in M. Maeda, et al., al., Chem. Lett., 1805-1808(1994) and B. A. Connolly, Nucleic Acids Res., 13, 4484(1985).

If the electroconductive substrate is made of glassy carbon electrode, the glassy carbon electrode is oxidized by potassium permanganate to produce a carboxylic acid group on the electrode. The carboxylic acid group on the electrode forms an amide bonding with DNA fragment so that the DNA fragment is fixed onto the substrate. See K.M. Millan, et al., Analytical Chemistry, 65, 2317-2323 (1993).

Probe molecules such as DNA fragments can be fixed to the substrate, initially, in the form of hybrid DNA fragments. For instance, the hybrid DNA fragments are combined with a mercapto group at their 5'- or 3'-terminals (preferably 5'-terminals) of their single fragment, and are brought into contact with a gold electrode, so that the hybrid DNA fragments are fixed on the electrode. The hybrid DNA fragment fixed on the electrode is then processed to dissociate a single fragment having no mercapto group, so that the desired DNA chip is produced.

The covalent bond between the DNA fragment and the substrate can be formed using an amino group, an aldehyde group, a mercapto group, or a biotin molecule which is attached to the DNA fragment.

Otherwise, probe molecules can be synthesized on the substrate by a known method.

The DNA fragment having a reactive group on one end can be fixed onto a substrate by spotting onto the substrate an aqueous solution containing the DNA fragment. The aqueous solution preferably contains the DNA fragment in a concentration of several pM to several mM. The volume for the spotting generally is in the range of 1 to 100 nL, preferably 1 to 10 nL. The aqueous solution may contain a viscosity increasing additive such as sucrose, polyethylene glycol, or glycerol. The spotting can be made manually or utilizing a commercially available spotter. The spotted solution is then kept on the substrate at a predetermined temperature for several hours (namely, incubation), whereby the DNA fragment is fixed onto the substrate by covalent bonding. After the incubation is complete, free DNA fragment (which is not fixed onto the substrate) is preferably washed out.

The DNA fragment is preferably fixed onto the substrate in an amount of 10⁻²⁰ to 10⁻¹² mol./mm² of the surface area of the substrate. The amount of the fixed DNA fragment can be determined by means of HPLC (high performance liquid chromatography) or other analytical apparatuses.

### [Hybridization]

The hybridization can be performed essentially in the same manner as that employed in various assay procedures utilizing the conventional DNA chip.

When the electrochemical analysis is performed, an electrochemically active molecule, specifically, an electrochemically active thread intercalator (namely, an electroconductive intercalator), is preferably employed for insertion into a hybrid formed by the DNA fragment and a sample nucleic acid on the electroconductive substrate. The intercalator assists easy flowing of electric current from or to the substrate along the formed hybrid structure. The electrochemical thread intercalator can be present when hybridization takes place. Otherwise, the thread intercalator can be brought into contact with a previously formed hybrid structure. In the latter case, a free nucleic acid which is not hybridized with the fixed DNA fragment is preferably removed from the substrate by washing with a mixture of a surfactant (preferably sodium dodecylsulfate) and a buffer (preferably a citrate buffer) in advance of the contact with the intercalator. The intercalator is preferably brought into contact with the hybrid in an aqueous solution at a concentration of 10 nM to 10 mM.

The hybridization is preferably performed at a temperature between room temperature and approximately 70°C, for 0.5 to 20 hours.

### [Electroconductive Intercalator]

The electroconductive intercalators favorably employed for the electrochemical analysis of nucleic acids are already known. A representative example of the intercalator is a thread intercalator having an electroconductive group at one end or both ends. The intercalator having the electroconductive group preferably has an oxidative-reductive activity. The oxidative-reductive activity can be imparted to the thread intercalator by incorporating into the intercalator a ferrocene group, a catechol amine group, a metal bipyridine complex group, a metal phenathroline complex group, or a viologen group. The intercalator moiety preferably comprises a naphthaleneimide moiety, an anthracene moiety, or an anthraquinone moiety. Preferred electroconductive intercalator is a ferrocene-containing naphthalene diimide compound [NDIFc₂-1, which is prepared from carboxylic acid ester of N-hydroxysuccinimide and a corresponding amine compound, see S. Takenaka et al., J. Chem. Soc. Commun., 1111 (1998)] : (NDIFc₂-1)

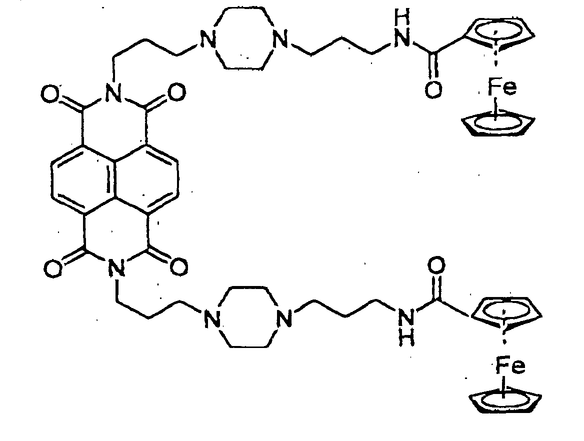

A ferrocene-containing naphthalene diimide derivative having the following formula is also preferably employed:

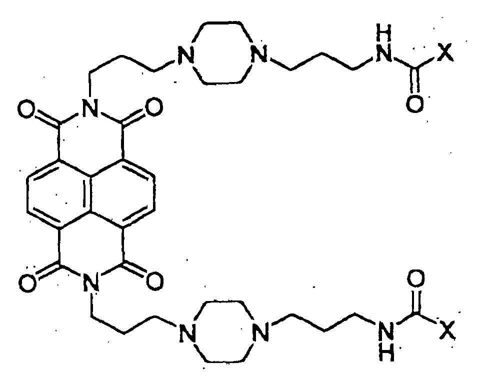

In the above-illustrated formula: X is one of the following ferrocene derivative groups:

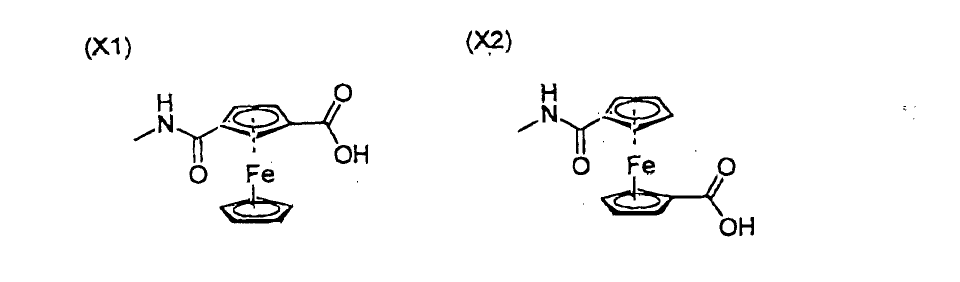

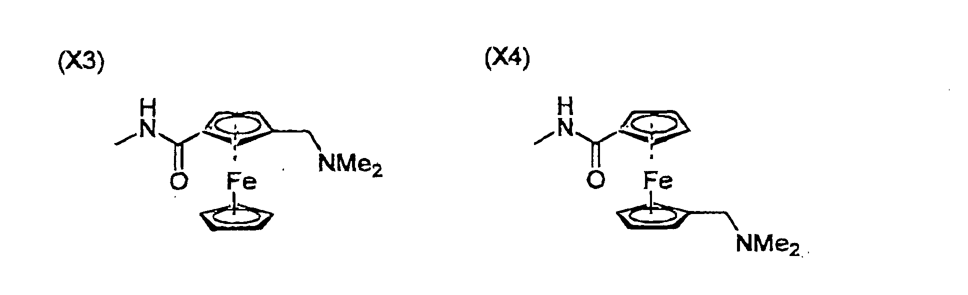

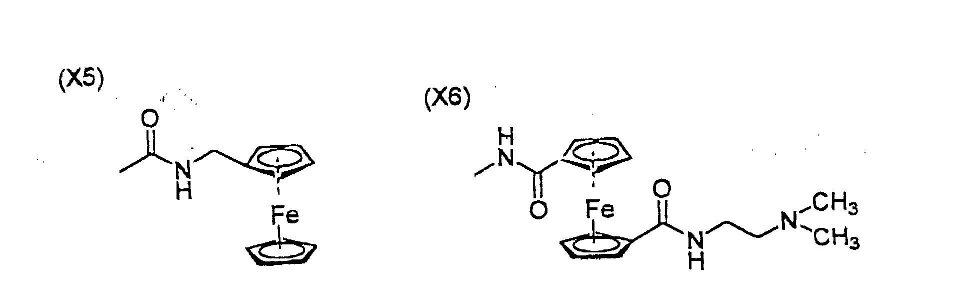

The thread intercalator having an electroconductive group comprises not only the oxidative-reductive active moiety and the intercalator moiety but also a linker moiety placed between these moieties. The 1,4-dipropylpiperazinyl group of the formula is an example of the linker moiety. The piperazinyl group can be replaced with an quaternary imino group. An intercalator of the below-illustrated formula which has a quaternary imino group always is cationic regardless of pH condition. This means that the intercalator is firmly fixed to the DNA hybrid and PNA hybrid. Accordingly, it is favorably employed in the invention. Particularly, the intercalator having a quaternary imino group is preferred in the use in combination with the PNA chip. The linker can be an N-alkyl group having 1 to 6 carbon atoms (e.g., methyl, ethyl, or n-propyl). The oxidative-reductive potential of the ferrocene moiety of the intercalator varies depending upon the nature of the linker moiety.

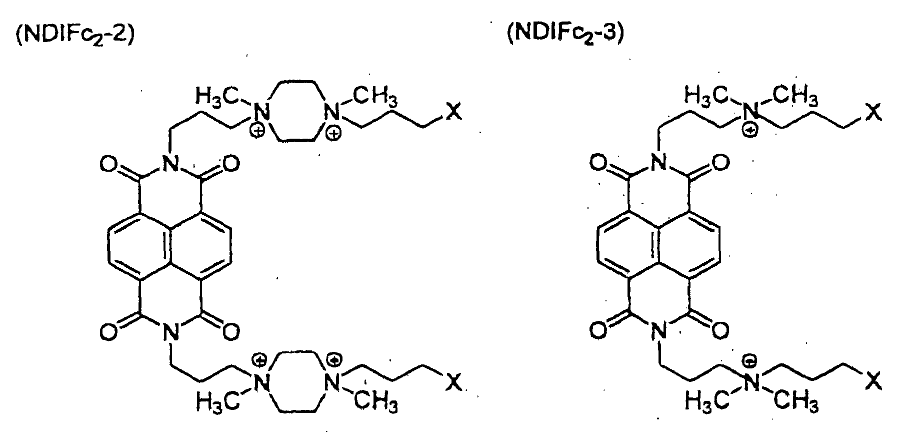

The electroconductive intercalators in the electrochemical detection of DNA fragment samples can have the formula (1), particularly the formula (2):

Ea - La - X - Lb - Eb (1)

Ea - L1a - L2a - X - L2b - L1b - Eb (2)

In the formulas (1) and (2), X represents a divalent cyclic group which may have one or more substituents.

The divalent cyclic group preferably is a plane cyclic group. Examples of the divalent cyclic groups include a naphthalene diimide group having two bonding sites at its two nitrogen atoms, an anthracene group having two bonding sites at 2- and 6-positions or 1- and 5- positions (preferably 2- and 6-positions), an anthraquinone group having two bonding sites in the same manner as in the anthracene group, a fluorene group having two bonding sites at 2- and 6-positions, a biphenylene group having two bonding sites at 2- and 6-positions, a phenantholene group having two bonding sites at 2- and 7-positions, and a pyrene group having two bonding sites at 2- and 7-positions. Preferred is a naphthalene diimide group having two bondings at the nitrogen atoms. The substituent can be a halogen atom (e.g., F, Cl, or Br), or an alkyl group having 1 to 6 carbon atoms such as methyl, ethyl, or n-propyl.

In the formula (1), each of La and Lb independently is a group which does not form a conjugated system in combination with the conjugated system of each of Ea and Eb and at least one of which has a site imparting water solubility to the compound or a site that is convertible into a site imparting water solubility to the compound. The site that is convertible into a site imparting water solubility to the compound means such site that it can be converted into a site imparting water solubility to the compound, for instance, by contact with an aqueous acidic solution such as an aqueous sulfuric acid. For instance, an imino group having a methyl substituent can be converted into a site having a sulfate group by contact with sulfuric acid. Thus formed site having a sulfate group imparts to the compound a necessary water solubility. The site can have an electric charge.

The water solubility is required for the compound in the case that the compound functions in an aqueous medium as the threading intercalator.

Each of La and Lb preferably has a hydrocarbyl group (which may have one or more substituents) on the side adjacent to Ea and Eb, respectively. The hydrocarbyl group corresponds to L1a and L1b of the formula (2) and further has a group having atomic elements other than carbon atoms on the side adjacent to X. The latter group corresponds to L2a and L2b of the formula (2). Accordingly, La and Lb are preferably represented by -L1a-L2a- and -L1b-L2b-, respectively.

Each of L1a and L1b preferably is an alkylene group having 1 to 6 carbon atoms or an alkenylene group having 2 to 6 carbon atoms, provided that each group may have one or more substituents. Each of L2a and L2b preferably is a linking group containing N, O or S.

Examples of the substituents for L1a and L1b include hydroxyl, halogen, carboxyl, amino, cyano, nitro, formyl, formylamino, alkyl having 1 to 6 carbon atoms, alkylamino having 1 to 6 carbon atoms, halogenated alkyl having 1 to 6 carbon atoms, cycloalkylamono having 5 to 7 carbon atoms, dialkylamino having 2 to 12 carbon atoms, aryl having 6 to 12 carbon atoms, aralkyl having 7 to 18 carbon atoms which contains alkyl of 1-6 carbon atoms, aralkylamino having 7 to 18 carbon atoms which contains alkyl of 1-6 carbon atoms, alkanoyl having 2 to 7 carbon atoms, alkanoylamino having 2 to 7 carbon atoms, N-alkanoyl-N-alkylamino having 3 to 10 carbon atoms, aminocarbonyl, alkoxycarbonyl having 2 to 7 carbon atoms, heterocyclic ring having 2 to 10 carbo atoms which has 1 to 4 hetero atoms such as S, N, or O, and aryl having 6 to 12 carbon atoms in its ring structure which may have 1 to 5 substituents such as alkyl of 1-6 carbon atoms, alkoxy of 1-6 carbon atoms, or halogen. The number of the substituents preferably is in the range of 1 to 12, more preferably 1 to 3, when the main chain is an alkylene group having 1 to 6 carbon atoms. The number of the substituents preferably is in the range of 1 to 10, preferably 1 to 3, when the main chain is an alkenylene group having 2 to 6 carbon atoms.

Each of L2a and L2b preferably is a linking group containing one or more groups such as an amino bonding, an ester bonding, an ether bonding, a thioether bonding, a diimide bonding, a thiodiimide bonding, a thioamide bonding, an imino bonding, a carbonyl bending, a thiocarbonyl bonding, or 1,4-piperazinyl bonding, any bonding possibly having one or more substituents. Each of L2a and L2b preferably contains -NHCO- or -CONH-.

Examples of the substituents for L2a and L2b include alkyl having 1 to 3 carbon atoms (e,g., methyl or ethyl), acyl having 2 to 4 carbon atoms (e.g., acetyl), aryl having 6 to 20 carbon atoms (e.g., phenyl or naphthyl), and aralkyl having 7 to 23 carbon atoms which has alkyl of 1-3 carbon atoms (e.g., benzyl).

When L2a or L2b contains an imino bonding, the imino bonding preferably contains a methyl substituent. Accordingly, each of L2a and L2b preferably is N-methyl-di(n-propylenyl)imino or 1,4-di(n-propylenyl)piperazinyl. Most preferred is N-methyl-di(n-propylenyl)imino.

Each of Ea and Eb has oxidation-reduction activity so that each has electroconductivity. It is preferred that each of Ea and Eb independently is a metallocene moiety, a 2,2'-bipyridine complex moiety, a cyclobutadiene moiety, a cyclopentadiene moiety, a 1,10-phenanthroline moiety, a triphenylphosphine moiety, a cathecol amine moiety, and a biologen moiety. Any moieties may have one or more substituents. Preferred are ferrocene moieties which may have one or more substituents. Examples of the substituted ferrocene moieties are illustrated below.

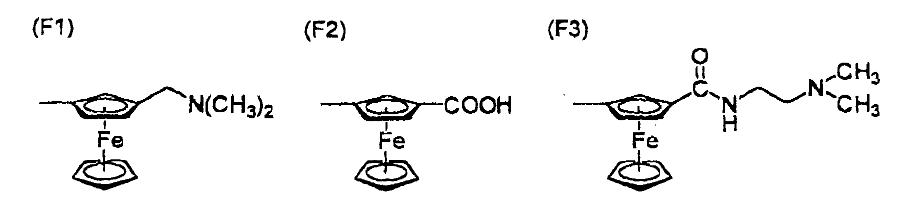

In the above-illustrated substituted ferrocene moieties, the substituent may be present in other positions on the cyclopentadienyl group.

The above-mentioned electroconductive intercalator can be prepared by a process similar to the process described in the aforementioned Japanese Patent Provisional Publication No. H9-288080.

### [Fluorescent Intercalator]

The fluorescent intercalator (i.e., fluorescent compound-labeled intercalator) is described below.

The fluorescent intercalator preferably is watersoluble and preferably has the following formula:

F - La - X

in which F is a fluorescent moiety, X is a cyclic group, and La is a linking group, in which at least one of X and La has a site imparting water solubility to the compound or a site that is convertible into a site imparting water solubility to the compound.

In the formula, X preferably has a substituent group represented by the formula of -Lb-Z, in which Z is a non-fluorescent moiety and Lb is a linking group. The fluorescent moiety generally emits a fluorescence in the wavelength region of 400 to 700 nm, preferably 400 to 550 nm.

A preferred example of the fluorescent moiety is a moiety having an acridine group, an indocyanine group, or an azaindoleninecyanine group. Also preferred is a groove binder group which has a quaternary cation in its molecular structure. The groove binder shows strong interaction with the anion of phosphate ester moiety of DNA fragment attached to a probe molecule of a DNA chip.

Representative examples of indocyanine groups, or azaindoleninecyanine groups, acridine groups, and groove binder group are illustrated below (A is oxygen atom or sulfur atom):

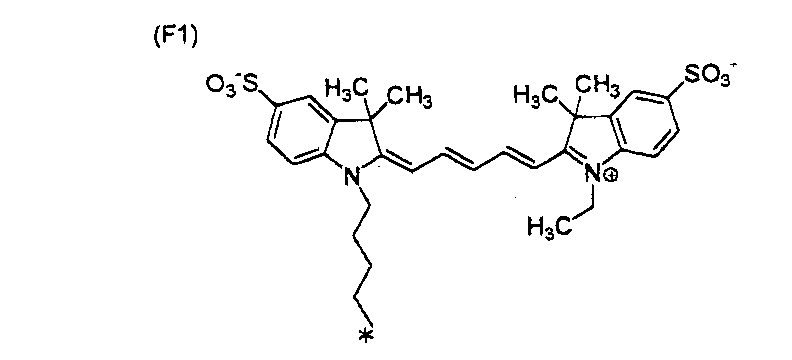

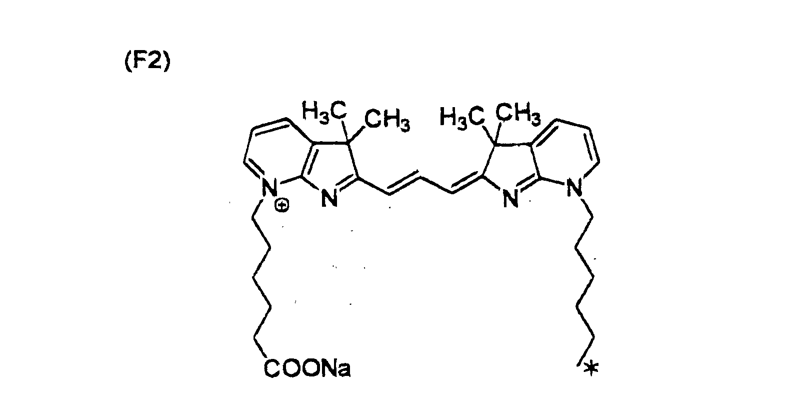

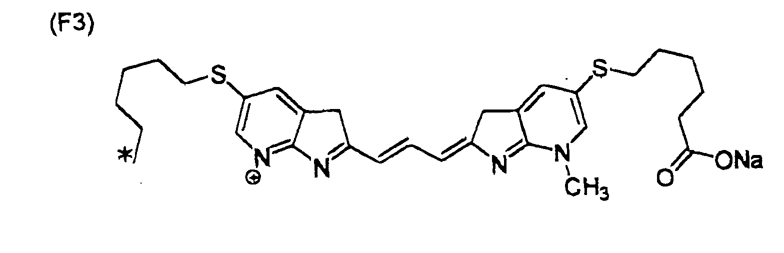

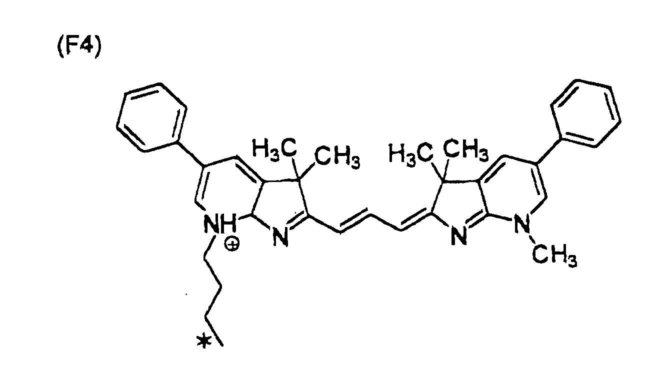

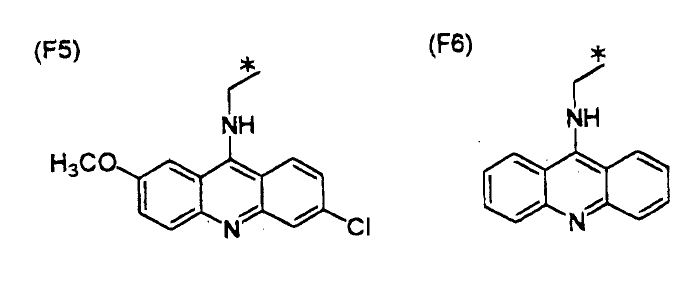

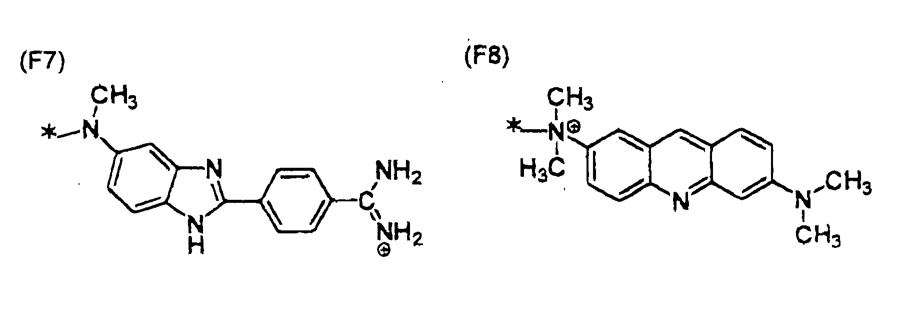

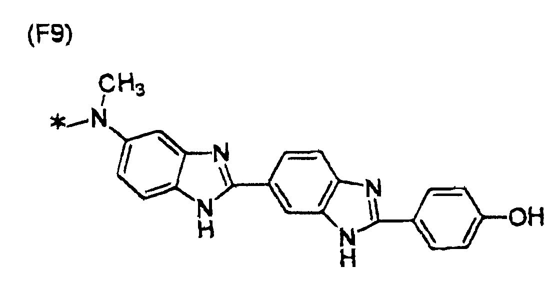

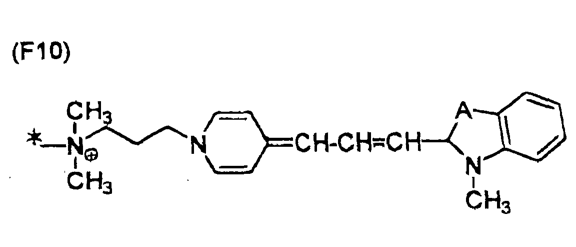

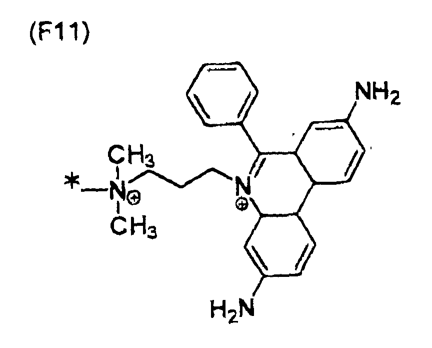

In the formula, X represents a cyclic group which may have one or more substituents. The cyclic group preferably is a plane cyclic group.

Examples of the cyclic groups include aromatic group having condensed aromatic rings such as a naphthalene diimide group having a bonding site at its nitrogen atom, an anthracene group having a bonding site at 1-, 2-, 5-, or 6-position (preferably 2- or 6-position), an anthraquinone group having a bonding site in the same manner as in the anthracene group, a fluorene group having a bonding site at 2- or 6-position, a biphenylene group having a bonding site at 2- or 6-position, a phenantholene group having a bonding site at 2- or 7-position, and a pyrene group having a bonding site at 2- or 7-position. Preferred is a naphthalene diimide group, an anthraquinone group, or an anthracene group.

The substituent can be a halogen atom (e.g., F, C1, or Br), or an alkyl group having 1 to 6 carbon atoms such as methyl, ethyl, or n-propyl.

Representative examples of X are illustrated below, in which the asterisk means a possible bonding site:

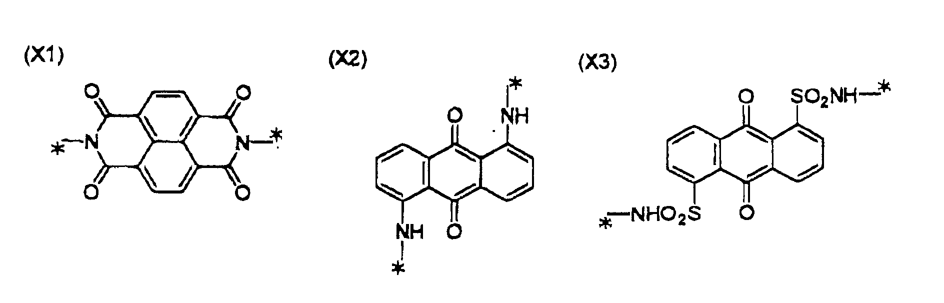

In the formula, it is preferred that La, i.e., linking group, has no aromatic group, and preferably contains one or more of an imino group, a 1,4-piperazinyl group, -O-, -S-, -CONH-, and -SO₂-.

La preferably contains a hydrocarbyl group (which may have one or more substituents) such as an alkylene group having 1 to 6 carbon atoms or an alkenylene group having 2 to 6 carbon atoms.

Examples of the substituents for La include hydroxyl, halogen, carboxyl, amino, cyano, nitro, formyl, formylamino, alkyl having 1 to 6 carbon atoms, alkylamino having 1 to 6 carbon atoms, halogenated alkyl having 1 to 6 carbon atoms, cycloalkylamono having 5 to 7 carbon atoms, dialkylamino having 2 to 12 carbon atoms, aryl having 6 to 12 carbon atoms, aralkyl having 7 to 18 carbon atoms which contains alkyl of 1-6 carbon atoms, aralkylamino having 7 to 18 carbon atoms which contains alkyl of 1-6 carbon atoms, alkanoyl having 2 to 7 carbon atoms, alkanoylamino having 2 to 7 carbon atoms, N-alkanoyl-N-alkylamino having 3 to 10 carbon atoms, aminocarbonyl, alkoxycarbonyl having 2 to 7 carbon atoms, heterocyclic ring having 2 to 10 carbon atoms which has 1 to 4 hetero atoms such as S, N, or O, and aryl having 6 to 12 carbon atoms in its ring structure which may have 1 to 5 substituents such as alkyl of 1-6 carbon atoms, alkoxy of 1-6 carbon atoms, or halogen. The number of the substituents preferably is in the range of 1 to 12, more preferably 1 to 3, when the main chain is an alkylene group having 1 to 6 carbon atoms. The number of the substituents preferably is in the range of 1 to 10, preferably 1 to 3, when the main chain is an alkenylene group having 2 to 6 carbon atoms.

La may contain one or more groups such as an amino bonding, an ester bonding, an ether bonding, a thioether bonding, a diimide bonding, a thiodiimide bonding, a thioamide bonding, an imino bonding, a carbonyl bonding, a thiocarbonyl bonding, or 1,4-piperazinyl bonding, any bonding possibly having one or more substituents.

The main chain of La (which means a chain along the shortest connection route from F to X) preferably contains 2 to 50 atoms, more preferably 5 to 30.

La preferably contains a site imparting water solubility to the compound or a site that is convertible into a site imparting water solubility to the compound. The site that is convertible into a site imparting water solubility to the compound means such site that it can be converted into a site imparting water solubility to the compound, for instance, by contact with an aqueous acidic solution such as an aqueous sulfuric acid. For instance, an imino group having a methyl substituent can be converted into a site having a sulfate group by contact with sulfuric acid. Thus formed site having a sulfate group imparts to the compound a necessary water solubility. The site can have an electric charge.

The water solubility can be introduced by producing the intercalator compound in the form of a water soluble salt, such as hydrochloride, sulfate, carbonate, phosphate, hydrobromide, hydroiodide, acetate, oxalate, malonate, succinate, maleate, fumarate, lactate, malate, citrate, tartrate, methanesulfonate, sodium salt, potassium salt, calcium salt, magnesium salt, pyridinium salt, ammonium salt, triethylamine salt, or ethanol amine salt.

The water solubility is required for the compound in the case that the compound functions in an aqueous medium as the intercalator.

In the formula, X can have a substituent group represented by -Lb-Z in which Z is a non-fluorescent moiety, preferably a non-fluorescent, non-aromatic moiety, which can contain oxygen, nitrogen and/or sulfur. More preferably, Z is a non-fluorescent moiety, preferably a non-fluorescent, non-aromatic moiety, which can contain an amino group, a carboxyl group, a sulfonic acid group, a sulfinic acid group, a sulfenic acid group, a hydrazino group, a carbamoyl group, a hydroxyl group, an imino group, and/or a mercapto group.

Lb is essentially the same as or similar to La of the formula. The resulting main chain of La-X-Lb preferably contains 10 to 100 atoms, which are counted along the shortest connection route from F to Z.

The fluorescent intercalator can be prepared in a manner similar to that described in Japanese Patent Provisional Publication 9-288080.

Other known fluorescent intercalators such as those described in Bull. Chem. Soc. Jpn., 72, 327-337(1999) are also employed.

### [Sample Nucleic Acid Fragment]

As is described before, the test method of the invention is preferably employed for detecting an abnormal gene fragment having one or more different bases from its corresponding normal gene fragment. Accordingly, the normal gene fragment or the abnormal gene fragment is preferably employed as the sample nucleic acid. It is preferred that the aknormal gene fragment is used as the sample nucleic acid.

The abnormal gene fragment can be defective and different from the corresponding normal gene fragment. Examples of the abnormal gene fragments include a gene fragment in which one or more base units are replaced with different base units, a gene fragment in which one or more base units are missing from the normal gene fragment, and a gene fragment in which one or more base units are added to the normal gene fragment.

The abnormal gene fragment is generally obtained from a living body having the double stranded abnormal gene. The double stranded abnormal gene is cleaved and treated in the conventional manner to give a single stranded abnormal gene fragment for performing the detection of the invention. The single stranded abnormal gene fragment can be chemically synthesized.

### [Detection of Partly Complementary Sample Nucleic Acid]

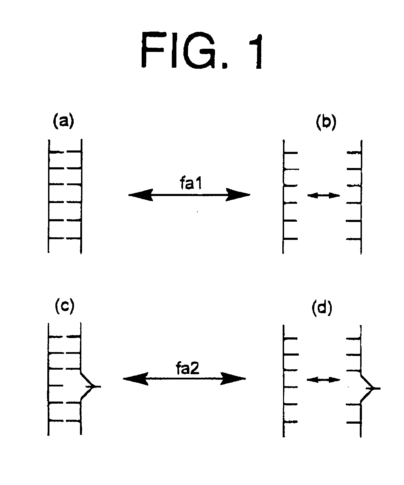

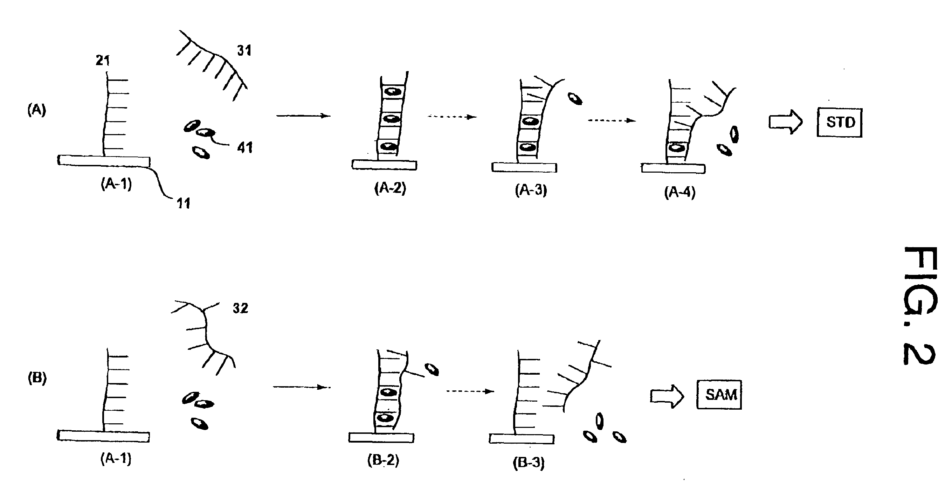

A method of the invention for testing a sample nucleic acid to judge whether the nucleic acid sample is partly complementary or fully complementary to the probe molecule in its specific base sequence is described by referring to Fig. 1 and Fig. 2.

In Fig. 1, the full-match hybridized structure (a) is formed from a combination (b) of a nucleic acid and a probe molecule under specific conditions fa1 or a nucleic acid is released from the full-match hybridized structure (a) under specific conditions fa1. The mis-match hybridized structure (c) is formed from a combination (d) of a nucleic acid and a probe molecule under specific conditions fa2 or a nucleic acid is released from the mis-match hybridized structure (d) under specific conditions fa2. The test method of the invention is based on detection of fa1 and fa2, and comparison of fa2 with fa1.

In Fig. 2, the test method comprising formation of a hybridized double-stranded structure and disengagement of a nucleic acid fragment from the structure is illustrated.

In the procedure (A), a DNA chip (A-1, which is composed of a substrate 11 and a probe molecule 21 fixed onto the substrate) is brought into contact with a reference nucleic acid 31 having a base sequence which is fully complementary to the probe molecule 21, in an aqueous medium, in the presence of a labeled intercalator 41. By the contact, the reference nucleic acid is combined with the probe molecule on the substrate. The labeled-intercalator is intercalated into the hybridized double-stranded structure, to form a reference nucleic acid complex (A-2).

The reference nucleic acid complex (A-2) is then placed in an aqueous solution, and the temperature of the aqueous solution is gradually increased. In the course of the temperature increase, the reference nucleic acid as well as the intercalator are released from the complex (A-2), as is illustrated under (A-3). The release advances further to release almost of the reference nucleic acid as well as the intercalator, as illustrated under (A-4). The advance of the release is continuously or intermittently examined by detecting the quantity of label remaining on the substrate. The data detected on the release of the reference nucleic acid as well as the labeled intercalator are recorded as STD (STAndard release data).

In the procedure (B), a partly complementary nucleic acid 32 is employed in place of the reference nucleic acid 31. A mis-mismatch structure is formed, as is illustrated under (B-2). The release of the partly complementary nucleic acid is then examined under the same conditions as employed in the procedure (A). The data of the remaining label detected on the release of the partly complementary nucleic acid as well as the labeled intercalator are recorded as SAM (SAMple release data).

STD and SAM are compared with each other, and it can be determined that the sample 32 is not a fully complementary (i.e., partly complementary) nucleic acid.

The process of formation of a full-match double stranded structure or a mis-match double stranded structure can be watched by examining the increase of quantity of label on the substrate from (A-4) to (A-2) and the quantity of the label from (B-3) to (B-2).

The variation of physical or chemical surrounding conditions can be applied to the aqueous medium by a variety of methods. Representative variations are increase or decrease of the temperature of the aqueous medium or increase or decrease of electrophoretic force applied to the nucleic acid complex or to the probe molecule.

The variation of temperature is generally made between 4 to 100°C, preferably 5 to 45°C, more preferably 15 to 45°C.

The variation of electrophoretic force can be applied to the complex, for instance, by applying varying electric gradient (potential) between the substrate (electrode) of DNA chip and a counter electrode (i.e., opposite electrode) which is also placed in contact with the aqueous medium. If the detection is performed using a fluorescent intercalator and using no electrode, a pair of electrodes are placed in the vicinity of the nucleic acid complex or the probe molecule to produce the electrophoretic force.

The variation of physical or chemical surrounding conditions can be applied to the aqueous medium by varying the ionic strength of the aqueous medium, varying the pH value of the aqueous medium, adding an organic solvent to the aqueous medium in a varying amount, or adding urea to the aqueous medium in varying amounts.

The variation of ionic strength is preferably performed by varying the amount of electrolyte (e.g., potassium chloride) to be added to the aqueous medium between 0 to 0.2M, preferably 0.1 to 0.9M, more preferably 0.3 to 0.7M.

The aqueous medium can be an aqueous buffer solution such as a citrate buffer, a phosphate buffer, a borate buffer, a Tris buffer, a Good buffer, a buffer solution containing an electrolytic material, or an aqueous organic acid containing dimethylsulfoxide which does not disturb the hybridization.

### [Detection of Label on Substrate]

The electroconductive label attached to the substrate by the formation of hybrid structure containing an electroconductive intercalator can be detected by measuring an electric current flowing from the electroconductive substrate to a counter-electrode (or vice versa) along the hybrid structure. The measurement of electric current can be performed by any of known methods such as cyclic voltamography (CV), differential pulse voltamography (DPD) and potentiostat. The differential pulse voltamography is most preferred.

The fluorescent label attached to the substrate by the formation of hybrid structure containing a fluorescent intercalator can be detected by measuring a fluorescence strength on the substrate, for instance, by fluorometry using a florescent laser scanner or a chilled CCD.

The present invention is further described by the following examples.

### [Example 1]

### (1) Preparation of DNA chip

A gold electrode (surface area: 2 mm²) was immersed in an aqueous 2N sodium hydroxide solution for one hour, washed with super pure water, and immersed in conc. nitric acid. The nitric acid was stirred for 15 min. The gold electrode was then washed with super pure water and dried. On thus treated gold electrode was spotted 1.0 µL of an aqueous solution containing 20 mers of adenine having a mercaptohexyl group at its 5'-terminal (HS-dA₂₀, in an amount of 10 pico mol./*µ*L), and the electrode was allowed to stand for 2 hours. The electrode was washed with super pure water to give a DNA chip. The preparation of HS-dA₂₀ was made in the manner described in Japanese Patent Provisional Publication No. 9-288080.

### (2) Masking of DNA chip

Onto the DNA chip prepared in (1) above was spotted 1 µL of an aqueous 2-mercaptoethanol solution (1 mM). The spotted solution was covered and allowed to stand for 2 hours. The DNA chip was then washed with super pure water.

### (3) Preparation of reference nucleic acid

A reference nucleic acid (dT₂₀) was prepared in the manner described in the above-mentioned patent publication.

### (4) Hybridization on DNA chip

One *µ*L of an aqueous solution containing 20 pico mol./*µ*L of dT₂₀ prepared in (3) above was spotted on the DNA chip, and the spotted chip was allowed to stand at 20°C for 30 min, for incubation.

### (5) Electroconductive intercalator

A ferrocene-containing naphthalene diimide intercalator illustrated below was employed.

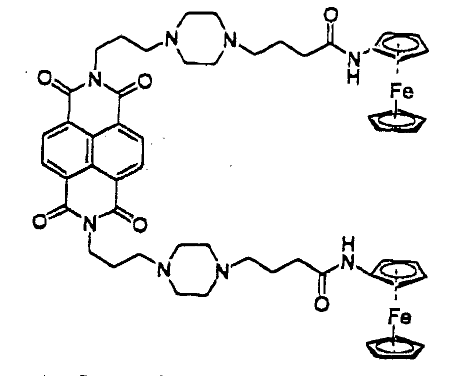

### (6) Measurement of electric current

An aqueous electrolytic solution [mixture of aqueous 0,1 M acetic acid/potassium acetate solution (pH 5.6) and aqueous 0.1 M potassium chloride solution] containing 50 *µ*M of the ferrocene-containing naphthalene diimide intercalator was placed in a thermostat cell. In the aqueous electrolytic solution were placed tri-electrodes composed of the DNA chip (i.e., working electrode), a platinum electrode (counter electrode or opposite electrode), and a silver/silver chloride referential electrode), and the temperature of the cell was varied between 15°C and 45°C. At the same time, differential pulse voltamography (DPV) was performed to determine a peal value at 460 mV.

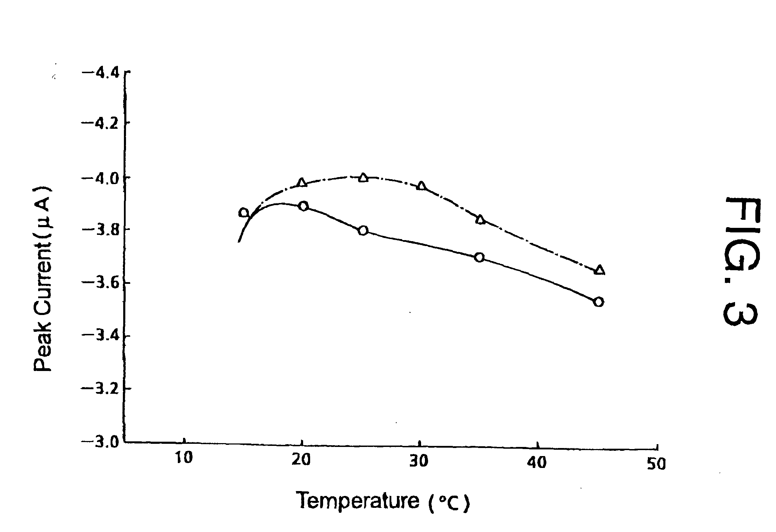

Fig. 3 shows the voltamography data for the full-match hybrid structure (dA₂₀/dT₂₀) by way of variation of peak current value, which is given by the curve connecting the blank triangles.

The peak current value at 20°C was determined using an aqueous solution containing no reference nucleic acid, for comparison. The corresponding peak current value given in the aforementioned procedure using an aqueous solution containing the reference nucleic acid was shifted by 37.3%.

The aforementioned procedures of (1) to (6) were repeated using a partly complementary sample oligonucleotide (dT₈dA₄dT₈) in place of the fully complementary dT₂₀.

Fig. 3 shows the voltamography data for the mis-match hybrid structure (dA₂₀/dT₈dA₄dT₈) by way of variation of peak current value, which is given by the curve connecting the circles. The peak current value at 20°C was also determined using an aqueous solution containing no reference nucleic acid, for comparison. The corresponding peak current value given in the above-mentioned procedure using an aqueous solution containing the reference nucleic acid was shifted by 15.1%.

Fig. 3 indicates that the peak current value varies sharply in the temperature range of 30 to 40°C in the case of the full-match hybrid structure (dA₂₀/dT₂₀), while the peak current value varies sharply in the temperature range of 20 to 30°C in the case of the mis-match hybrid structure (dA₂₀/dT₈dA₄dT₈). This means that the mis-match structure can be detected by the above-described method.

### [Example 2]

### (1) Preparation of DNA chip

A gold electrode (surface area: 2.25 mm²) was immersed in an aqueous 2N sodium hydroxide solution for one hour, washed with super pure water, and immersed in conc. nitric acid. The nitric acid was stirred for 15 min. The gold electrode was then washed with super pure water and dried. On thus treated gold electrode was spotted 2.0 *µ*L of an aqueous solution containing 20 mers of adenine having a mercaptohexyl group at its 5'-terminal (HS-dA₂₀, in an amount of 100 pico mol./µL), and the electrode was allowed to stand for one hour. The electrode was washed with super pure water to give a DNA chip.

The aqueous solution containing HS-dA₂₀ and an aqueous solution recovered after the spotting were subjected to high performance liquid chromatography (HPLC) to measure change of the peak assigned to HS-dA₂₀, whereby determining the amount of the fixed HS-dA₂₀. It was confirmed that 20 pico mol. of HS-dA₂₀ was fixed onto the gold electrode.

### (2) Masking of DNA chip

Onto the DNA chip prepared in (1) above was spotted 1 *µ*L of an aqueous 2-mercaptoethanol solution (1 mM). The spotted solution was covered and allowed to stand for 2 hours. The DNA chip was then washed with super pure water.

### (3) Preparation of reference and sample nucleic acids

A reference nucleic acid (dT₂₀) and a sample nucleic acid (dT₉dA₁dT₁₀) were prepared in the manner described in the above-mentioned patent publication.

### (4) Hybridization on DNA chip

2 *µ*L of an aqueous solution containing 70 pico mol./*µ*L of dT₂₀ or dT₉dA₁dT₁₀ prepared in (3) above was spotted on the DNA chip, and the spotted chip was covered to keep the spotted solution from evaporation and allowed to stand at 25°C for 20 min, for incubation. After the incubation was complete, the electrode was washed with an aqueous solution (pH 7.0) of 0.1M sodium dihydrogen phosphate/disodium hydzogen phosphate to remove un-fixed nucleic acid.

### (5) Electroconductive intercalator

A ferrocene-containing naphthalene diimide intercalator illustrated below was employed.

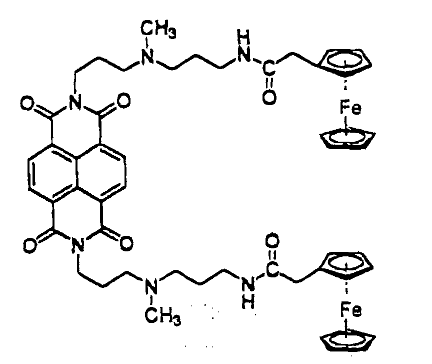

### (6) Measurement of electric current

An aqueous electrolytic solution [mixture of aqueous 0.1 M acetic acid/potassium acetate solution (pH 5.6) and aqueous 0.1 M potassium chloride solution] containing 50 *µ*M of the ferrocene-containing naphthalene diimide intercalator was placed in a thermostat cell. In the aqueous electrolytic solution were placed tri-electrodes composed of the DNA chip (i.e., working electrode), a platinum electrode (counter electrode or opposite electrode), and a silver/silver chloride referential electrode), and the temperature of the cell was varied between 15°C, 25°C, 35°C and 45°C. At the same time, differential pulse voltamography (DPV) was performed to determine a peal value. The measurement was performed at a pulse amplitude of 50 mV, a pulse width of 50 mS, and a scanning rate of 100 mV/sec.

The results indicate that the peak current value varies sharply in the temperature range of 35 to 45°C in the case of the full-match hybrid structure (dA₂₀/dT₂₀), while the peak current value varies sharply in the temperature range of 25 to 35°C in the case of the mis-match hybrid structure (dA₂₀/dT₉dA₁dT₁₀). This means that the mis-match structure can be detected by the above-described method.

### [Example 3]

### (1) to (5)

Performed in the same manner as in (1) to (5) of Example 2.

### (6) Measurement of electric current

An aqueous electrolytic solution [aqueous 0.1 M acetic acid/potassium acetate solution (pH 5.6)] containing 50 *µ*M of the ferrocene-containing naphthalene diimide intercalator was placed in a thermostat cell at 25°C. In the aqueous electrolytic solution were placed tri-electrodes composed of the DNA chip (i.e., working electrode), a platinum electrode (counter electrode or opposite electrode), and a silver/silver chloride referential electrode). The electric gradient between the working electrode and the opposite electrode was varied from 0.0 V to 1.6 V stepwise at 0.2 V intervals. At the same time, differential pulse voltamography (DPV) was performed. The measurement was performed at a pulse amplitude of 50 mV, a pulse width of 50 mS, and a scanning rate of 100 mV/sec.

The results indicate that the peak current value decreases sharply at an electric gradient of 1.2 V in the case of the full-match hybrid structure (dA₂₀/dT₂₀), while the peak current value decreases sharply at an electric gradient of 0.8 V in the case of the mis-match hybrid structure (dA₂₀/dT₉dA₁dT₁₀). This means that the mis-match structure can be detected by the above-described method.

### [Example 4]

### (1) to (5)

Performed in the same manner as in (1) to (5) of Example 2.

### (6) Measurement of electric current

An aqueous electrolytic solution [aqueous 0.1 M acetic acid/potassium chloride solution (pH 5.6)] containing 50 *µ*M of the ferrocene-containing naphthalene diimide intercalator was placed in a thermostat cell at 25°C. In the aqueous electrolytic solution were placed tri-electrodes composed of the DNA chip (i.e., working electrode), a platinum electrode (counter electrode or opposite electrode), and a silver/silver chloride referential electrode). The concentration of potassium chloride in the electrolytic solution was varied between 0.6M to 0.0M at 0.1M intervals. At the same time, differential pulse voltamography (DPV) was performed. The measurement was performed at a pulse amplitude of 50 mV, a pulse width of 50 mS, and a scanning rate of 100 mV/sec.

The results indicate that the peak current value decreases sharply at a potassium chloride concentration range of 0.1M to 0.0M in the case of the full-match hybrid structure (dA₂₀/dT₂₀), while the peak current value decreases sharply at a potassium chloride concentration range of 0.2M to 0.1M in the case of the mis-match hybrid structure (dA₂₀/dT₉dA₁dT₁₀). This means that the mis-match structure can be detected by the above-described method.

## Claims

1. A method for testing complementation of nucleic acid fragment which comprises the steps of:
bringing a sample nucleic acid complex which comprises a double-stranded nucleic acid structure and a labeled intercalator intercalated therein, in which the double-stranded nucleic acid structure has been produced by contact of a sample nucleic acid fragment with a probe molecule fixed to a solid carrier in the presence of an aqueous medium, the probe molecule being selected from the group consisting of a nucleic acid or a nucleic acid derivative, into contact with an aqueous medium;
applying variation of physical or chemical surrounding conditions to the latter aqueous medium, to cause disengagement of the sample nucleic acid fragment and the intercalator from the nucleic acid complex and simultaneously measuring decrease of quantity of the label on the solid carrier, so that stability of the sample nucleic acid fragment of the complex is determined; and
comparing the stability determined above with reference stability data which are separately obtained by determination of stability of a reference nucleic acid fragment in a reference nucleic acid complex comprising a reference double-stranded nucleic acid structure and the labeled intercalator intercalated therein in which the reference double-stranded nucleic acid structure is produced by contact of the reference nucleic acid fragment with the probe molecule, the reference nucleic acid fragment being determined in complementation thereof with the probe molecule.

2. The method of claim 1, wherein the variation of physical or chemical surrounding conditions is variation of a temperature of the aqueous medium.

3. The method of claim 1, wherein the variation of physical or chemical surrounding conditions is variation of electrophoretic potential.

4. The method of claim 1, wherein the variation of physical or chemical surrounding conditions is variation of ionic strength in the aqueous medium.

5. The method of claim 1, wherein the labeled intercalator is an intercalator having an electroconductive label.

6. The method of claim 1, wherein the labeled intercalator is an intercalator having a fluorescent label.

7. The method of claim 1, wherein the probe molecule contains a chain of a base sequence comprising at least three predetermined base units in series.

8. The method of claim 7, wherein the reference nucleic acid fragment contains a chain of a base sequence comprising at least three base units in series which are fully complementary to the chain of the probe molecule.

9. The method of claim 1, wherein the reference stability data are obtained by a step which is identical to the first step of claim 1, except for replacing the sample nucleic acid complex with the reference nucleic acid complex.

10. The method of claim 1, wherein the probe molecule is selected from the group consisting of oligonucleotide, polynucleotide, and peptide nucleic acid.

11. A method for testing complementation of nucleic acid fragment which comprises the steps of:
bringing a sample nucleic acid fragment into contact with a probe molecule fixed to a solid carrier in the presence of an aqueous medium and a labeled intercalator to produce on the solid carrier a sample nucleic acid complex comprising a double-stranded nucleic acid structure and the labeled intercalator intercalated therein, the probe molecule being selected from the group consisting of a nucleic acid or a nucleic acid derivative, while applying variation of physical or chemical surrounding conditions to the aqueous medium, so that stability of the sample nucleic acid fragment in the complex is determined; and
comparing the stability determined above with reference stability data which are separately obtained by determination of stability of a reference nucleic acid fragment in a reference nucleic acid complex comprising a reference double-stranded nucleic acid structure and the labeled intercalator intercalated therein in which the reference double-stranded nucleic acid structure is produced by contact of the reference nucleic acid fragment with the probe molecule, the reference nucleic acid fragment being determined in complementation thereof with the probe molecule.

12. The method of claim 11, wherein the variation of physical or chemical surrounding conditions is variation of a temperature of the aqueous medium.

13. The method of claim 11, wherein the variation of physical or chemical surrounding conditions is variation of electrophoretic potential.

14. The method of claim 11, wherein the variation of physical or chemical surrounding conditions is variation of ionic strength in the aqueous medium.

15. The method of claim 11, wherein the labeled intercalator is an intercalator having an electroconductive label.

16. The method of claim 11, wherein the labeled intercalator is an intercalator having a fluorescent label.

17. The method of claim 11, wherein the probe molecule contains a chain of a base sequence comprising at least three predetermined base units in series.

18. The method of claim 17, wherein the reference nucleic acid fragment contains a chain of a base sequence comprising at least three base units in series which are fully complementary to the chain of the probe molecule.

19. The method of claim 11, wherein the reference stability data are obtained by a step which is identical to the first step of claim 11, except for replacing the sample nucleic acid complex with the reference nucleic acid complex.

20. The method of claim 11, wherein the probe molecule is selected from the group consisting of oligonucleotide, polynucleotide, and peptide nucleic acid.
